# EUROPEAN PATENT APPLICATION

(11) **EP 3 135 769 A1**
(43) Date of publication of application: **01.03.2017**
(21) Application number: 15182605.4
(22) Date of filing: 26.08.2015
(51) Int. Cl.: C12Q 1/68

(54) **KITS AND METHODS FOR EXTRACTING RNA**

(71) Applicant: Qiagen GmbH, 40724 Hilden (DE)
(72) Inventor: Wende, Andy, 40724 Hilden (DE); Werner, Sabine, 40724 Hilden (DE)
(74) Representative: Friedrich, Rainer

(57) **Abstract**

The present invention provides novel kits and methods for extracting RNA from an RNA-containing sample. The kits and methods of the invention employ a lysis buffer and a heat-metastable protease which together achieve an improved efficiency of RNA extraction from biological samples, including from samples with a high level of RNAse activity. The kits and methods of the invention are therefore particularly useful for extracting RNA from complex samples such as blood, plasma and swabs, said lysis buffer comprises a zwitterionic buffering agent, a chelating agent, a basic amino acid, a cyclic amino acid, a calcium salt, a non-ionic non-denaturing detergent, and polyethylene glycol (PEG).

SEQUENCE LISTING

<110> Qiagen GmbH

<120> KITS AND METHODS FOR EXTRACTING RNA

<130> QIA15999EP

<160> 3

<170> BiSSAP 1.3

<210> 1
<211> 23
<212> DNA
<213> Artificial Sequence

<220>
<223> Fwd primer for Artus influenza amplification

<400> 1
cttctgatcc gcatagtgac gac                                              23

<210> 2
<211> 22
<212> DNA
<213> Artificial Sequence

<220>
<223> Rev primer for Artus influenza amplification

<400> 2
aacggtcatt cgcctccagc ag                                               22

<210> 3
<211> 23
<212> DNA
<213> Artificial Sequence

<220>
<223> Probe primer for Artus influenza amplification

<400> 3
taggggatgg taacgacgaa gca                                              23

## Description

### FIELD OF THE INVENTION

The present invention relates to kits and methods for extracting RNA from an RNA-containing sample. The kits and methods of the invention employ a particular lysis buffer and a heat-metastable protease, which together result in an improved efficiency of RNA extraction from biological samples. The kits and methods of the invention are capable of extracting RNA from biological samples which contain a high level of RNAse activity and are therefore particularly useful in the fields of RNA analysis and diagnostics.

### BACKGROUND OF THE INVENTION

The determination of RNA levels is of great importance in the fields of molecular biology and cell biology as well as in the field of diagnostics, particularly for the analysis of gene expression. The activities of genes can be determined directly by the analysis of the RNA, in particular the mRNA in cells. The quantitative analysis of transcript samples (mRNA samples) in cells by modern molecular-biological methods enables the recognition, for example, of wrongly expressed genes whereby metabolic disorders, infections or the formation of cancer can be recognised. The analysis of RNA is also used for the direct detection of infectious agents, such as viruses, bacteria, fungi etc. RNA analysis methods include principally reverse transcriptase-polymerase chain reaction (RT-PCR), quantitative PCR (qPCR), Northern blotting, and the like. These methods depend on a consistent supply of high quality RNA. Therefore, effective and reliable procedures to extract intact RNA from biological samples are essential.

Biological samples, such as blood, sputum, urine, tissue etc., contain cells or organisms. In order to extract nucleic acid, including RNA, from the cells or organisms of the sample, the cells or organisms must be disintegrated, so that the contents are not present within but outside of the cells or organisms. The disintegration of cells or organisms is called lysis; the disintegrated cells or organisms are also called lysates.

An early method for isolating total RNA is disclosed in Chirgwin, J. M. et al: Biochem, 18:5294-5299 (1979). In that procedure, RNA-containing tissue is homogenized in a solution containing guanidinium thiocyanate, sodium citrate and 2-mercaptoethanol. The homogenate is then centrifuged and the supernatant decanted and mixed with acetic acid and absolute ethanol. Overnight storage at -20°C precipitates the RNA and it is recovered in pellet form after centrifugation. The pellet is re-dissolved in a buffered guanidine hydrochloride solution and re-precipitated by adding acetic acid and ethanol. The last step is repeated and the isolated RNA is recovered in pellet form. Clearly, this method is rather laborious and time-consuming.

Subsequently, many prior art methods of isolating nucleic acid from complex starting materials like whole blood, blood serum, urine or faeces usually comprise lysis of biological material by a detergent in the presence of protein degrading enzymes, followed by several rounds of extraction with organic solvents, e.g., phenol and/or chloroform, ethanol precipitation and dialysis of the nucleic acids (see, for example US 5,681,946). However, these methods are also rather laborious and time-consuming. Furthermore, the relatively large number of steps required to purify nucleic acid from such starting materials increases the risk of transmission of nucleic acid from sample to sample in the simultaneous processing of several clinical samples. When the nucleic acid is isolated for the subsequent detection of the presence of nucleic acid of, e.g., a pathogen such as a virus or a bacterium, by means of a nucleic acid amplification method, the increased risk of such a transmission of nucleic acid between different samples may cause false positive results which are a serious drawback.

In another method, phenol and guanidine procedures were combined, resulting in a method of total RNA isolation that can be completed in about 3 hours (US 4,843,155). This method was improved upon in US 5,346,994, which allows for completion of the RNA isolation in about 1 hour. Those phenol/guanidine methods involve the use of a monophasic solution of phenol and guanidine isothiocyanate, commercially available as the reagent Trizol^{®} (Invitrogen Corp.). Unfortunately, it was found that total RNA samples extracted from clinical isolates using Trizol^{®} or hot phenol methods can experience unacceptable levels of high molecular weight DNA contamination, as determined by ethidium bromide gel electrophoresis. US 8,062,845 discloses a similar method of isolating RNA using Trizol^{®} whereby the contaminating genomic DNA is minimized, although not eliminated. However, this method still involves the use of organic solvents and alcohols, which are hazardous, even in small amounts. Furthermore, it involves time-consuming RNA precipitation.

Other RNA extraction methods take advantage of the fact that nucleic acids bind to acidic surfaces in the presence of chaotropic salt solutions. This was originally described for diatomaceous earth and silicon dioxide particles (US 5,234,809). A process for isolating nucleic acid from a nucleic acid-containing starting material using chaotropic salt solutions involves mixing the starting material with the chaotropic substance and a nucleic acid binding solid phase (e.g. silica particles capable of binding the nucleic acid in the presence of a chaotropic substance), separating the solid phase with the nucleic acid bound thereto from the liquid, washing the solid phase-nucleic acid complexes which are obtained, and if required, eluting the nucleic acid from said complexes (see, for example, WO 98/59076). Alternatively, a combination of chaotropes and alcohols can be used (WO 95/01359). When the chaotropes (and alcohols) are removed from the system, the negatively charged surfaces allow an efficient elution, i.e, dissociation of nucleic acids from the solid phase into the water suspension. Many customary lysis buffers for extraction of nucleic acids now contain chaotropic ion mixtures as salts. However, both chaotropes and alcohols are potentially hazardous chemicals and they can also easily influence downstream analysis, thereby reducing to some extent the user acceptance of the method. Furthermore, the sophisticated purification procedures that often need to be employed with these methods can be very time-consuming. Another potential problem of this method is that, once protein impurities inherent to the sample are washed away, the buffer conditions may allow a renaturation of proteins such as nucleases which, in the case of RNases, reduces RNA yield.

In general, the RNA extraction efficiency of prior art methods is relatively low, particularly for complex samples. In addition, swabs or complex samples such as urine often contain low numbers of cells; therefore, extraction levels and/or the purity of RNA obtained by prior art methods may not always be sufficient and/or appropriate for downstream applications. However, there is presently no method for extraction of RNA from complex samples (or crude samples), which works without chaotropic salt conditions or a sophisticated purification procedure. Clearly, a simple and effective method for RNA extraction from a wide range of biological samples is desirable. Considering the high-throughput nature of many RNA analyses, it is also highly desirable that RNA extraction methods are fast and uncomplicated, e.g. capable of being repeatedly performed direct from cell lysates without the need for sophisticated purification.

Thus, it is the object of the present invention to provide kits and methods for improved extraction of RNA from a biological sample in a simple, rapid, safe and effective procedure.

### SUMMARY OF THE INVENTION

It was surprisingly found that the particular composition of the lysis buffer according to the kits and methods of the invention, together with a heat-metastable protease, achieves an effective and reliable extraction of RNA from biological target material, even from complex samples. A particular advantage of the kits and methods of the invention is that they employ a lysis buffer which does not require any chaotropic salt for RNA extraction. Thus, the kits and methods of the invention are safe and, moreover, they do not require a sophisticated purification step, thereby providing an uncomplicated and fast procedure for RNA extraction.

The present invention relates to kits for extracting RNA comprising a lysis buffer and a heat-metastable protease. Also provided are methods for extracting RNA which employ a lysis buffer and a heat-metastable protease. The kits and methods of the invention are capable of extracting RNA from a range of biological samples, including complex samples which may contain a number of impurities and/or may be rich in RNAse activity (e.g. blood samples or swabs).

Accordingly, in a first aspect the invention provides a kit for extracting RNA from an RNA-containing sample, the kit comprising:
(a) a lysis buffer comprising a zwitterionic buffering agent, a chelating agent, a basic amino acid, a cyclic amino acid, a calcium salt, a non-ionic non-denaturing detergent, and polyethylene glycol (PEG); and
(b) a heat-metastable protease.

In an alternative aspect, the invention provides a kit for extracting RNA from an RNA-containing sample, the kit comprising a lysis buffer comprising a zwitterionic buffering agent, a chelating agent, a basic amino acid, a cyclic amino acid, a calcium salt, a non-ionic non-denaturing detergent, polyethylene glycol (PEG) and a heat-metastable protease.

According to certain embodiments, the kits may further comprise one or more of the following:
(c) magnetic particles for performing sample enrichment and/or reverse purification of RNA;
(d) reagents (e.g. wash buffers, elution buffers) for performing sample enrichment, and/or column purification or reverse purification of RNA;
(e) deoxyribonuclease; and/or
(f) an instruction manual detailing how to use the kit to extract RNA.

According to certain embodiments, the kits may further comprise at least two of, at least three of, or all four of (c), (d), (e) and (f) above.

In one embodiment, the lysis buffer contained in the kits comprises HEPES or MOPS as the zwitterionic buffering agent, EDTA or EGTA as the chelating agent, arginine as the basic amino acid, proline as the cyclic amino acid, CaCl₂ as the calcium salt, one of Nonidet P40, IGEPAL CA-630, Triton or Tween20 as the non-ionic non-denaturing detergent, and polyethylene glycol (PEG) with a molecular weight between 4000 and 8000.

In another embodiment, the lysis buffer contained in the kits comprises:
(a) HEPES pH 7.5 to 8.5;
(b) disodium EDTA;
(c) arginine;
(d) proline;
(e) CaCl₂;
(f) Nonidet P40 or IGEPAL CA-630; and
(g) PEG of molecular weight around 6000.

In a specific embodiment, the lysis buffer contained in the kits comprises:
(a) HEPES pH 7.5 to 8.5 at a concentration between 1 mM and 30 mM;
(b) disodium EDTA at a concentration between 0.005 mM and 0.3 mM;
(c) arginine at a concentration between 1 mM and 20 mM;
(d) proline at a concentration between 1 mM and 20 mM;
(e) CaCl₂ at a concentration between 0.05 mM and 1.5 mM;
(f) Nonidet P40 or IGEPAL CA-630 at a concentration between 0.01% (v/v) and 0.5% (v/v); and
(g) PEG of molecular weight around 6000 at a concentration between 0.01 % (v/v) and 1 % (v/v).

In one embodiment, the RNA-containing sample for which the kits are used to extract RNA from is a swab, optionally a throat swab or a nasal swab. In another embodiment, the RNA-containing sample comprises whole blood, blood plasma or blood serum. In a further embodiment, the RNA-containing sample comprises cultured cells, optionally infected with virus particles. In certain embodiments, the RNA-containing sample is a swab, optionally a throat swab or a nasal swab; and it comprises whole blood, blood plasma or blood serum. In another embodiment, the RNA-containing sample is a nasal wash or a nasal aspirate. In another embodiment, the RNA-containing sample is a broncho-alveolar lavage (BAL). In another embodiment, the RNA-containing sample is a tissue sample, such as epithelial, buccal, cervical, intestinal, pancreas, muscle, kidney, heart, lung, brain, bone, prostate, skin, hair, or a removed tumour tissue sample. In another embodiment, the RNA-containing sample is a bodily fluid, such as sputum, saliva, tears, cerebrospinal fluid (CSF), urine, stool, semen, or sweat. In yet another embodiment, the RNA-containing sample is a liquor sample, for example, from a lumbar puncture.

In one embodiment, the heat-metastable protease is added to or is present in the lysis buffer in an amount which is sufficient to inactivate RNAses in the mixture that forms when contacting the sample with the lysis buffer, but which does not impair amplification enzymes which are added to the mixture in downstream applications.

In a preferred embodiment, the concentration of the heat-metastable protease in the lysis buffer is between 0.2 and 4.0 mAU/ml.

In one embodiment, the heat-metastable protease is subtilisin or proteinase K. In a preferred embodiment, the heat-metastable protease is subtilisin.

In certain embodiments where the kits comprise deoxyribonuclease, the deoxyribonuclease may also be added to the lysis buffer prior to contacting the RNA-containing sample with said lysis buffer.

In a second aspect, the invention provides a method of extracting RNA from an RNA-containing sample, the method comprising the following steps:
(i) adding a heat-metastable protease to a lysis buffer, wherein the lysis buffer comprises a zwitterionic buffering agent, a chelating agent, a basic amino acid, a cyclic amino acid, a calcium salt, a non-ionic non-denaturing detergent, and polyethylene glycol (PEG);
(ii) contacting the RNA-containing sample with the lysis buffer containing the heat-metastable protease of step (i) to form a mixture;
(iii) optionally heating the mixture to at least 60°C, at least 70°C or at least 80°C; and
(iv) optionally recovering the RNA from the mixture.

In an alternative aspect, the invention provides a method of extracting RNA from an RNA-containing sample, the method comprising the following steps:
(i) contacting the RNA-containing sample with a lysis buffer to form a mixture, wherein the lysis buffer comprises a zwitterionic buffering agent, a chelating agent, a basic amino acid, a cyclic amino acid, a calcium salt, a non-ionic non-denaturing detergent, polyethylene glycol (PEG), and a heat-metastable protease;
(ii) optionally heating the mixture to at least 60°C, at least 70°C or at least 80°C; and
(iii) optionally recovering the RNA from the mixture.

In one embodiment, the lysis buffer employed in the methods comprises HEPES or MOPS as the zwitterionic buffering agent, EDTA or EGTA as the chelating agent, arginine as the basic amino acid, proline as the cyclic amino acid, CaCl₂ as the calcium salt, one of Nonidet P40, IGEPAL CA-630, Triton or Tween20 as the non-ionic non-denaturing detergent, and polyethylene glycol (PEG) with a molecular weight between 4000 and 8000.

In another embodiment, the lysis buffer employed in the methods comprises:
(a) HEPES pH 7.5 to 8.5;
(b) disodium EDTA;
(c) arginine;
(d) proline;
(e) CaCl₂;
(f) Nonidet P40 or IGEPAL CA-630; and
(g) PEG of molecular weight around 6000.

In a specific embodiment, the lysis buffer employed in the methods comprises:
(a) HEPES pH 7.5 to 8.5 at a concentration between 1 mM and 30 mM;
(b) disodium EDTA at a concentration between 0.005 mM and 0.3 mM;
(c) arginine at a concentration between 1 mM and 20 mM;
(d) proline at a concentration between 1 mM and 20 mM;
(e) CaCl₂ at a concentration between 0.05 mM and 1.5 mM;
(f) Nonidet P40 or IGEPAL CA-630 at a concentration between 0.01% (v/v) and 0.5% (v/v); and
(g) PEG of molecular weight around 6000 at a concentration between 0.01 % (v/v) and 1% (v/v).

In one embodiment of the methods, the RNA-containing sample is a swab, optionally a throat swab or a nasal swab. In another embodiment, the RNA-containing sample comprises whole blood, blood plasma or blood serum. In a further embodiment, the RNA-containing sample comprises cultured cells, optionally infected with virus particles. In certain embodiments, the RNA-containing sample is a swab, optionally a throat swab or a nasal swab; and it comprises whole blood, blood plasma or blood serum. In another embodiment, the RNA-containing sample is a nasal wash or a nasal aspirate. In another embodiment, the RNA-containing sample is a broncho-alveolar lavage (BAL). In another embodiment, the RNA-containing sample is a tissue sample, such as epithelial, buccal, cervical, intestinal, pancreas, muscle, kidney, heart, lung, brain, bone, prostate, skin, hair, or a removed tumour tissue sample. In another embodiment, the RNA-containing sample is a bodily fluid, such as sputum, saliva, tears, cerebrospinal fluid (CSF), urine, stool, semen, or sweat. In yet another embodiment, the RNA-containing sample is a liquor sample, for example, from a lumbar puncture.

In certain embodiments of the methods, for example, when the RNA-containing sample comprises cells, whole blood, blood plasma, blood serum, a nasal wash or nasal aspirate, BAL or a bodily fluid, prior to the lysis step of the method the sample may be enriched for the presence of biological target material (e.g. cells, viruses, bacteria). In a further embodiment, the enrichment procedure employs magnetic particles which bind to said biological target material in the sample. The magnetic particles bound to biological target material can be subsequently removed from the sample by applying an external magnetic force, e.g. a magnetic stand. The isolated magnetic particles bound to biological target material can then be contacted with the lysis buffer according to the respective method and then the method can proceed as described above. In a preferred embodiment, the enrichment procedure does not employ a precipitating agent.

In one embodiment, the heat-metastable protease is added to or is present in the lysis buffer in step (i) of the methods in an amount which is sufficient to inactivate RNAses in the mixture that forms when contacting the sample with the lysis buffer, but which does not impair amplification enzymes which are added to the mixture in downstream applications.

In a preferred embodiment, the concentration of the heat-metastable protease in the lysis buffer is between 0.2 and 4.0 mAU/ml.

In one embodiment, the heat-metastable protease is subtilisin or proteinase K. In a preferred embodiment, the heat-metastable protease is subtilisin.

In certain embodiments, deoxyribonuclease may also be added to the lysis buffer in step (i) of the methods prior to contacting the RNA-containing sample with said lysis buffer.

In one embodiment of the methods, the RNA is recovered from the mixture by column purification.

In an alternative embodiment of the methods, the RNA is recovered from the mixture by reverse purification. In a further embodiment, the reverse purification employs magnetic particles which bind to non-RNA components in the mixture. The magnetic particles bound to non-RNA components can be subsequently removed from the mixture by applying an external magnetic force, e.g. a magnetic stand.

### BRIEF DESCRIPTION OF THE DRAWINGS

**Figure 1****:** Ct-values from Phage fr-specific RT-qPCR using RNA extracts with various amounts of nasal swab matrix at different temperatures. As the percentage of nasal swab matrix increases in the sample, the Ct value increases (corresponding to decreasing amounts of RNA).
**Figure 2****:** Ct-values from Influenza A-specific RT-qPCR using RNA extracts prepared with varying amounts of heat-metastable protease in the lysis buffer. The graph shows that there is a range of heat-metastable protease concentration which provides an optimum (lower) Ct value.

### DETAILED DESCRIPTION OF THE INVENTION

### I. Definitions

In order that the present invention may be readily understood, several definitions of terms used in the course of the invention are set forth below.

The term *"lysis"* as used herein refers to the disruption, degradation and/or digestion of the biological sample. In a lysis step, nucleic acids can be released from cells or organisms or can be freed from other sample materials or components such as e.g. proteins.

The term *"RNA"*, as understood here, refers to ribonucleic acid and includes the naturally occurring and not naturally occurring types, i.e. any or all of mRNA, hnRNA, rRNA, tRNA, miRNA, and/or siRNA.

The term *"an RNA-containing sample"* as used herein is intended to mean any sample containing biological material. For example, the RNA-containing sample may comprise one or more of whole blood, blood plasma, blood serum, tissues (such as epithelial, buccal, cervical, intestinal, pancreas, muscle, kidney, heart, lung, brain, bone, prostate, skin, hair, or a removed tumour tissue sample), a nasal wash or a nasal aspirate, a broncho-alveolar lavage (BAL), a bodily fluid (such as sputum, saliva, tears, cerebrospinal fluid (CSF), urine, stool, semen, or sweat), a liquor sample (for example, from a lumbar puncture), cultured cells, cultured microorganisms, cultured viruses and materials containing cellular material, organisms or crude RNA.

The term *"heat-metastable protease"* as used herein means a protease which is stable, and therefore active, up to a certain temperature threshold. Beyond said temperature threshold, the protease exhibits reduced stability and is thereby inactivated. In the context of the present invention, the temperature threshold of the heat-metastable protease is generally 50-80°C. The heat-metastable protease in the lysis buffer of the invention can be any protease which has these characteristics. For a protease equivalent to subtilisin, the temperature threshold is around 60-70°C. For a protease equivalent to proteinase K, the temperature threshold is around 70-80°C. If proteins (enzymes) will later be added to the recovered RNA sample for downstream applications, the temperature of the heating step should be above that of the temperature threshold of the heat-metastable protease so that the protease is inactivated. In this regard, the heat inactivation temperature should be at least 10°C above the individual temperature threshold of the applied heat-metastable protease. The heat-metastable protease added to the lysis buffer according to the kits and methods of the invention is preferably subtilisin or proteinase K or an equivalent heat-metastable protease.

The term *"column purification"* in the context of the invention means a procedure that takes advantage of nucleic acid binding to a solid phase in a column in order to directly purify the nucleic acids. This method relies on the fact that nucleic acids will bind to the solid phase (e.g. anion exchange, silica) on a column under appropriate buffer, salt and pH conditions.

The term *"reverse purification"* in the context of the invention means a procedure that removes impurities and/or contaminants from the lysate or mixture in order to leave behind nucleic acids, thus indirectly purifying nucleic acids in said lysate or mixture. An example of reverse purification is the use of magnetic particles which bind to impurities and/or contaminants in the lysate or mixture and which can subsequently be removed from the mixture by applying an external magnetic force.

The term *"external magnetic force"* refers to the application of a magnetic field, typically through a device or apparatus, at least a part of which is magnetised. In the context of the invention, the external magnetic force is used for specifically directing the movement of magnetic particles. The magnetic force may be applied by a magnetic stand, for example.

The term *"AU"* stands for "Anson Units", a standard measurement of protease activity. One Anson unit is defined as the amount of enzyme that liberates Folin-positive amino acids and peptides, corresponding to 1 µmol tyrosine under assay conditions in 1 minute using haemoglobin as substrate (Anson M.M, J. Gen. Physiol., 22: 79, 1939).

Unless defined otherwise, all further technical and scientific terms used herein have the same meaning as commonly understood by one of ordinary skill in the art (e.g., in cell culture, molecular genetics, nucleic acid chemistry, hybridization techniques and biochemistry).

The terms *"of the invention", "in accordance with the invention",* or *"according to the invention"* as used herein are intended to refer to all aspects and embodiments of the invention described and/or claimed herein.

As used herein, the term *"comprising"* is to be construed as encompassing both *"including"* and *"consisting of"*, both meanings being specifically intended, and hence individually disclosed embodiments in accordance with the invention.

In practicing the present invention, many conventional techniques in molecular biology, microbiology, and recombinant DNA may be used. These techniques are well known and are explained in, for example, Current Protocols in Molecular Biology, Volumes I, II, and III, 1997 (F. M. Ausubel ed.); Sambrook et al., 1989, Molecular Cloning: A Laboratory Manual, Second Edition, Cold Spring Harbor Laboratory Press, Cold Spring Harbor, N.Y.; DNA Cloning: A Practical Approach, Volumes 1 and 11, 1985 (D. N. Glover ed.); Oligonucleotide Synthesis, 1984 (M. L. Gait ed.); Nucleic Acid Hybridization, 1985, (Hames and Higgins); Transcription and Translation, 1984 (Hames and Higgins eds.); Animal Cell Culture, 1986 (R. I. Freshney ed.); Immobilized Cells and Enzymes, 1986 (IRL Press); Perbal, 1984, A Practical Guide to Molecular Cloning; the series, Methods in Enzymology (Academic Press, Inc.); Gene Transfer Vectors for Mammalian Cells, 1987 (J. H. Miller and M. P. Calos eds., Cold Spring Harbor Laboratory); and Methods in Enzymology Vol. 154 and Vol. 155 (Wu and Grossman, and Wu, eds., respectively).

### II. Kits for RNA Extraction

In a first aspect, the invention provides a kit comprising a lysis buffer and a heat-metastable protease for extracting RNA from an RNA-containing sample. The lysis buffer of the kit comprises a zwitterionic buffering agent, a chelating agent, a basic amino acid, a cyclic amino acid, a calcium salt, a non-ionic non-denaturing detergent, and polyethylene glycol (PEG).

In an alternative aspect, the invention provides a kit for extracting RNA from an RNA-containing sample, said kit comprising a lysis buffer comprising a zwitterionic buffering agent, a chelating agent, a basic amino acid, a cyclic amino acid, a calcium salt, a non-ionic non-denaturing detergent, polyethylene glycol (PEG), and a heat-metastable protease.

The lysis buffer used in the kits of the invention may be a lysis buffer according to any of the embodiments described in detail in section IV of the present description and/or any of the embodiments claimed herein. In a preferred embodiment, the lysis buffer according to the kits of the invention does not comprise a chaotropic salt.

In certain embodiments, the heat-metastable protease contained in the kits is subtilisin or proteinase K, preferably subtilisin. Subtilisins are a family of serine proteases, i.e. enzymes with a serine residue in the active site, which have broad substrate specificities. Discussions of subtilisins, proteinase K and other proteases may be found, for example, in Genov et al., Int. J. Peptide Protein Res. 45: 391-400, 1995.

The heat-metastable protease contained in the kits can be present in the lysis buffer of the kit, but it is preferably separate from the lysis buffer, to be added to the lysis buffer prior to contacting it with the RNA-containing sample. The heat-metastable protease is inactivated by heating to at least 60°C. If the heat-metastable protease is subtilisin or an equivalent, the heat-metastable protease is preferably inactivated by heating to at least 70°C. If the heat-metastable protease is proteinase K or an equivalent, the heat-metastable protease is preferably inactivated by heating to at least 80°C. In one embodiment, the heat-metastable protease is added to / is present in the kits at an amount which is sufficient to inactivate RNAses in the mixture that forms when contacting the sample with the lysis buffer, but which, after heating, does not impair amplification enzymes which are added to the mixture in downstream applications. Preferably, the concentration of the heat-metastable protease in the lysis buffer is between 0.2 and 4.0 mAU/ml, most preferably between 1.0 and 3.0 mAU/ml.

In a preferred embodiment, the kit contains the heat-metastable protease in a lyophilized form. In a further embodiment, the kit additionally comprises a protease resuspension buffer to re-suspend lyophilized protease.

In one embodiment, the kits may be used for extracting RNA from an RNA-containing sample comprising cultured cells, optionally infected with virus particles. In another embodiment, the kits may be used for extracting RNA from a swab, optionally a throat swab or a nasal swab. In another embodiment, the kits may be used for extracting RNA from a sample which comprises whole blood, blood plasma or blood serum. In a further embodiment, the kits may be used for extracting RNA from a sample which comprises a swab and whole blood, blood plasma or blood serum. In another embodiment, the kits may be used for extracting RNA from a nasal wash or a nasal aspirate. In another embodiment, the kits may be used for extracting RNA from a broncho-alveolar lavage (BAL). In another embodiment, the kits may be used for extracting RNA from a tissue sample, such as epithelial, buccal, cervical, intestinal, pancreas, muscle, kidney, heart, lung, brain, bone, prostate, skin, hair, or a removed tumour tissue sample. In another embodiment, the kits may be used for extracting RNA from a bodily fluid, such as sputum, saliva, tears, cerebrospinal fluid (CSF), urine, stool, semen, or sweat. In yet another embodiment, the kits may be used for extracting RNA from a liquor sample, for example, from a lumbar puncture.

In one embodiment, the kits further comprise magnetic particles for performing sample enrichment and/or reverse purification of RNA from a lysate (mixture of lysis buffer and RNA-containing sample). The magnetic particles in the kits preferably have an anionic surface that is capable of binding amplification inhibitors, cells, debris and impurities. For this purpose, it is also within the scope of the present invention to functionalize the surface of the magnetic particles with appropriate functional groups. Accordingly, the magnetic particles may comprise or consist of a polymer capable of forming an anionic structure, e.g. a copolymer or terpolymer, a carboxylated polymer or polyester capable of forming an anionic structure. According to one embodiment, said polymer is a carboxylated polymer e.g. based on vinyl methyl ether, maleic anhydride, styrene, linear or branched alkenes or acrylic acid and its derivatives.

The particles may have an average size that is selected from a range 100 nm to 50 µm, preferably 400 nm to 30 µm, and more preferably 800 nm to 3.5 µm. Particles of the respective sizes and in particular of a smaller size such as 10 µm or less, 7.5 µm or less, preferably 5 µm or less, 2.5 µm or less or 1.5 µm or less are easy to handle and can be well re-suspended in the lysis mixture.

The magnetic particles can have superparamagnetic, paramagnetic, ferrimagnetic or ferromagnetic characteristics. Preferably, superparamagnetic particles are used. The magnetic particles may comprise a magnetic material that is incorporated in the particles and/or is associated with the particles. To avoid leaching of the magnetic material, the magnetic material is preferably completely encapsulated. The magnetic material may provide the core(s) of the particles, may be comprised in the core and/or may be applied onto the core of the particle.

In certain embodiments, the kits further comprise an enrichment buffer for use in performing sample enrichment with the magnetic particles contained in the kits. The enrichment buffer may, for example, be a buffer which comprises about 100mM sodium acetate/acetic acid pH 5.5 and about 75mM sodium chloride.

In another embodiment, the kits further comprise reagents (e.g. wash buffers, elution buffers) for performing sample enrichment, and/or column purification or reverse purification of RNA from a lysate.

In another embodiment, the kits further comprise deoxyribonuclease, preferably in lyophilized form. In a further embodiment, the kits comprise a resuspension buffer suitable for deoxyribonuclease. Alternatively, the deoxyribonuclease may simply be added to the lysis buffer of the kits.

In another embodiment, the kits further comprise carrier RNA, for example in lyophilized form. Carrier RNA enhances the binding of viral nucleic acids to solid phase (e.g. during column purification), especially if there are very few target molecules in the sample. In addition, relatively large amounts of carrier RNA reduce the chance of viral RNA degradation.

In a further embodiment, the kits comprise an internal control (DNA and/or RNA) in addition to the carrier RNA. The internal control DNA and/or RNA may be added together with the carrier RNA to the lysis buffer. For optimal purification efficiency, internal control molecules should be longer than 200 nucleotides, as smaller molecules may not be efficiently recovered.

In another embodiment, the kits may comprise an instruction manual detailing how to use the kits to extract RNA and/or how to perform each step of the extraction of RNA according to the methods of the invention.

In particular embodiments, the kits of the invention (comprising the lysis buffer and a heat-metastable protease) further comprise magnetic particles for performing sample enrichment and/or reverse purification of RNA, enrichment buffer, at least one wash buffer, at least one elution buffer, carrier RNA, internal controls, protease resuspension buffer, deoxyribonuclease, and an instruction manual.

In a preferred embodiment, the kits of the invention (comprising the lysis buffer and a heat-metastable protease) further comprise at least magnetic particles for performing sample enrichment and/or reverse purification of RNA.

The kits of the invention may be used to perform any of the methods described and/or claimed herein.

### III. Methods of RNA Extraction

In a second aspect, the present invention provides a method of extracting RNA from an RNA-containing sample, said method comprising the following steps:
(i) adding a heat-metastable protease to a lysis buffer, wherein the lysis buffer comprises a zwitterionic buffering agent, a chelating agent, a basic amino acid, a cyclic amino acid, a calcium salt, a non-ionic non-denaturing detergent, and polyethylene glycol (PEG)
(ii) contacting the RNA-containing sample with the lysis buffer containing the heat-metastable protease of step (i) to form a mixture;
(iii) optionally heating the mixture to at least 60°C, at least 70°C, or at least 80°C; and
(iv) optionally recovering the RNA from the mixture.

In an alternative aspect, the invention provides a method of extracting RNA from an RNA-containing sample, the method comprising the following steps:
(i) contacting the RNA-containing sample with a lysis buffer to form a mixture, wherein the lysis buffer comprises a zwitterionic buffering agent, a chelating agent, a basic amino acid, a cyclic amino acid, a calcium salt, a non-ionic non-denaturing detergent, polyethylene glycol (PEG), and a heat-metastable protease;
(ii) optionally heating the mixture to at least 60°C, at least 70°C or at least 80°C; and
(iii) optionally recovering the RNA from the mixture.

The lysis buffer used the methods of the invention may be a lysis buffer according to any of the embodiments described in detail in section IV of the present description and/or any of the embodiments claimed herein. In a preferred embodiment, the lysis buffer according to the methods of the invention does not comprise a chaotropic salt.

In step (i) of the methods, the heat-metastable protease is either added to the lysis buffer or it is already present in the lysis buffer. In one embodiment, the heat-metastable protease is added to / is present in the lysis buffer at an amount which is sufficient to inactivate RNAses in the mixture that forms when contacting the sample with the lysis buffer, but which does not impair amplification enzymes which are added to the mixture in downstream applications. Preferably, the concentration of the heat-metastable protease in the lysis buffer is between 0.2 and 4.0 mAU/ml, most preferably between 1.0 and 3.0 mAU/ml. In certain embodiments, the heat-metastable protease is subtilisin or proteinase K, preferably subtilisin.

One important advantage of using a protease that is heat-metastable is that it can be almost completely inactivated by heat incubation, e.g. around 60 °C and beyond, depending on the particular heat-metastable protease. This may be useful, for example, if the protease needs to be inactivated in order to perform downstream applications in the same reaction vessel as lysis. If the protease is used at higher concentrations (e.g. around 2.0 to around 4.0 mAU/ml), it may also increase the lysis efficiency of eukaryotic cells in the sample.

In certain embodiments of the methods, deoxyribonuclease may be added to the lysis buffer prior to contacting the sample with the lysis buffer in order to remove DNA from the sample/mixture. The deoxyribonuclease should be present in the lysis buffer/mixture at a concentration between 1 and 1000 U/ml. The removal of DNA may further improve the extraction of RNA and may also increase the efficiency of downstream applications.

The general protocol for extracting RNA according to the methods of the present invention is suitable for a variety of RNA-containing samples or materials. In one embodiment of the methods, the RNA-containing sample comprises cultured cells, optionally infected with virus particles. In another embodiment, the RNA-containing sample is a swab, such as a throat swab or a nasal swab. In a further embodiment, the RNA-containing sample comprises whole blood, blood plasma or blood serum. In certain embodiments, the RNA-containing sample is a swab, and it comprises whole blood, blood plasma or blood serum. In another embodiment, the RNA-containing sample is a nasal wash or a nasal aspirate. In another embodiment, the RNA-containing sample is a broncho-alveolar lavage (BAL). In another embodiment, the RNA-containing sample is a tissue sample, such as epithelial, buccal, cervical, intestinal, pancreas, muscle, kidney, heart, lung, brain, bone, prostate, skin, hair, or a removed tumour tissue sample. In another embodiment, the RNA-containing sample is a bodily fluid, such as sputum, saliva, tears, cerebrospinal fluid (CSF), urine, stool, semen, or sweat. In yet another embodiment, the RNA-containing sample is a liquor sample, for example, from a lumbar puncture.

Plasma or serum samples should be centrifuged after collection and can then generally be stored at 2-8°C for up to 6 hours. For long-term storage, freezing at -20°C or -80°C in aliquots is recommended. Frozen plasma or serum samples should not be thawed more than once. Repeated freeze-thawing leads to denaturation and precipitation of proteins, which may result in reduced yields of RNA extraction.

According to the methods of the invention, swab samples can undergo cell lysis and rapid nucleic acid extraction immediately. In the case of liquid samples, such as a nasal wash or nasal aspirate, urine, saliva, whole blood, serum, plasma, BAL, or samples comprising cultured cells, an enrichment step may be required prior to contacting the sample with the lysis buffer in order to enrich the sample for biological target material (e.g. cells, viruses, bacteria).

In certain embodiments, the enrichment procedure employs magnetic particles which bind to said biological target material in the sample. Magnetic particles are particularly beneficial because of the ease with which they can be separated from an associated liquid phase. The magnetic particles bound to biological target material can be subsequently removed from the sample by applying an external magnetic force, e.g. a magnetic stand. The isolated magnetic particles bound to biological target material can then be contacted with the lysis buffer according to the lysis step of the method. In a preferred embodiment, the enrichment procedure does not employ a precipitating agent.

For complex liquid matrices, such as whole blood, a wash step may also be implemented following biological target material enrichment but prior to lysis, for example by using a hypotonic washing buffer.

Swab samples are preferably directly lysed in a low volume (e.g. 200 µl to 1 ml) of lysis buffer before proceeding with the RNA extraction procedure. The same applies to biological target material that has been enriched prior to lysis.

When choosing a volume of lysis buffer for swab samples, the skilled person will appreciate that swabs retain different amounts of liquid depending on their composition; for example, woven swabs retain more liquid than flocked swabs. Elution of swabs in low volumes generally increases the concentration of target material. Likewise, the skilled person will appreciate that the volume of lysis buffer used for lysing cells which have been enriched from a culture solution may be adjusted depending on the density of the cell culture.

For more effective lysis, the sample may be incubated in the lysis buffer of the invention at an elevated temperature, i.e. any temperature between room temperature and 95°C. The efficiency of lysis is proportional to the temperature. However, if proteins (enzymes) will later be added to the mixture for downstream applications, the mixture must be heated to at least 10°C above the temperature threshold of the heat-metastable protease included in the lysis mixture. In this case, the mixture should generally be heated to at least 60°C to inactivate the heat-metastable protease. If the heat-metastable protease is subtilisin or an equivalent, the mixture should be heated to at least 70°C. If the heat-metastable protease is proteinase K or an equivalent, the mixture should be heated to at least 80°C.

Additionally or alternatively, more effective lysis may be achieved by incubating the sample in the lysis buffer for an extended period of time, i.e. for up to 1 minute, for up to 5 minutes, for up to 10 minutes or for up to 15 minutes, or longer. The period of time will depend on the sample. For example, for samples containing viruses and/or eukaryotic cells, heat incubation may be extended up to 15 minutes. Additionally, shaking during heat incubation can be used to improve heat transfer into the lysis buffer and sample mixture.

The lysis can further be carried out under mechanical effect or with enzymatic support. As a means for the mechanical support are considered mortars, the application of high pressure, narrow capillaries and the use of filter units. An enzymatic support of the lysis can for example be supported by the use of lysozymes, cellulases, pectinases, or additional proteases, whereby the degradation of the structure of the organism(s) in the sample is supported further.

In one embodiment of the methods, the RNA is recovered from the mixture by column purification. For example, the method may rely on the fact that RNA will bind to the solid phase (e.g. anion exchange, silica) on a column under appropriate conditions. The steps of separating the solid phase with the nucleic acid bound thereto from any liquid phase are intended to remove contaminants or undesired residuals in any liquid phase. Any further washing steps with any suitable composition(s) known to those skilled in the art (for instance water, chaotropic solutions, buffers, etc.) may be applied to wash the solid phase with the nucleic acid bound thereto. Any convenient separation steps known to those skilled in the art can be utilised in accordance with the present invention. Examples of such a purification method which cleans nucleic acids from a sample via a silica membrane are RNeasy^{®} or QIAamp^{®} kits, obtained from the company QIAGEN GmbH, Hilden, Germany.

In an alternative embodiment of the methods, the RNA is recovered from the mixture by reverse purification. In one embodiment, reverse purification is achieved by non-magnetic particles. The separation of non-magnetic particles may be carried out for example by centrifugation or filtration. In a preferred embodiment, reverse purification is achieved by magnetic particles, which bind to non-RNA components in the mixture. The magnetic particles bound to non-RNA components can be subsequently removed from the mixture by applying an external magnetic force, e.g. a magnetic stand or a permanent magnet. An RNA-containing liquid phase substantially free of contaminants/impurities remains, which liquid phase qualifies as "recovered RNA", and this RNA-containing liquid phase is already suitable for downstream applications, such as RT-PCR.

The magnetic particles used in the enrichment procedure and/or in reverse purification are described above in section II (kits of the invention). Systems for the removal of magnetic particles are well-known in the prior art and are also commercially available (e.g. QIASYMPHONY^{®}; QIAGEN GmbH, Hilden, Germany). The collected magnetic particles can then be processed further. Such systems are also well-known in the prior art and are also commercially available (e.g. BioRobot EZ1, QIAGEN GmbH, Hilden, Germany) and thus, do not need any detailed description here.

### IV. Lysis Buffer according to the Kits and the Methods of the Invention

The lysis buffer described in detail in this section is the lysis buffer which is contained in the kits of the invention for extracting RNA and which is employed by the methods of the invention for extracting RNA.

The lysis buffer according to the kits and methods of the invention comprises a zwitterionic buffering agent. This buffering agent should be capable of buffering the aqueous lysis composition (mixture) at physiological pH and preferably at a pH range that is compatible with the pH range that is required for performing the subsequent analytical method, such as e.g. an amplification reaction. It follows that the pH value of the mixture obtained by contacting lysis buffer with sample should be between about pH 5 and about pH 9, with a pH value between pH 7.5 to pH 8.5 being preferred. In one embodiment, the buffer substance is selected from the group consisting of MOPS and HEPES. Preferably, the lysis buffer of the invention contains HEPES at pH 7.5 to 8.5 as the zwitterionic buffering agent.

In one embodiment, the zwitterionic buffering agent is comprised in the lysis buffer according to the kits and methods of the invention in a concentration between 1 mM and 30 mM, preferably between 2 mM and 15 mM. In one embodiment, the zwitterionic buffering agent of the lysis buffer according to the kits and methods of the invention is HEPES with a pH value between 7.5 and 8.5 at a concentration between 1 mM and 30 mM. In a preferred embodiment, the zwitterionic buffering agent of the lysis buffer according to the kits and methods of the invention is HEPES with a pH value between 7.5 and 8.5 at a concentration between 2 mM and 15 mM. In a most preferred embodiment, the zwitterionic buffering agent of the lysis buffer according to the kits and methods of the invention is HEPES with a pH value between 7.5 and 8.5 at a concentration between 4 mM and 8 mM.

The lysis buffer according to the kits and methods of the invention comprises a chelating agent, which should be suitable for chelating divalent cations. The chelating agent helps to protect nucleic acids from degradation by nucleases and it helps to provide the 'salt' requirement to maintain the cell membrane and/or the cell nucleus at physiological salt conditions, to avoid osmotic disruption. Accordingly, the chelating agent may be chosen from any group of chelating agents which are compatible with biological samples, for example, from EDTAs, EGTAs, citrates and/or combinations thereof. In a preferred embodiment, the chelating agent is EDTA, more preferably disodium EDTA or the like. Using a chelating agent such as EDTA has the advantageous effect of DNase and RNase inhibition. In one embodiment, the chelating agent is present in the lysis buffer at a concentration between about 0.005 mM and about 0.3 mM, preferably between about 0.01 mM and about 0.1 mM. In one embodiment, the chelating agent in the lysis buffer is EDTA at a concentration between about 0.005 mM and about 0.3 mM. In a preferred embodiment, the chelating agent in the lysis buffer is EDTA at a concentration between 0.01 mM and 0.1 mM. In a most preferred embodiment, the chelating agent in the lysis buffer is EDTA at a concentration between about 0.03 mM and about 0.07 mM.

The lysis buffer according to the kits and methods of the invention comprises a basic amino acid and a cyclic amino acid. In one embodiment, the basic amino acid is arginine. In another embodiment, the cyclic amino acid is proline. In a preferred embodiment, the lysis buffer comprises both arginine and proline.

In one embodiment, the basic amino acid and a cyclic amino acid are each present in the lysis buffer at a concentration between about 1 mM and about 20 mM, preferably between about 2 mM and about 10 mM. In a further embodiment, the lysis buffer comprises between about 1 mM and about 20 mM arginine and between about 1 mM and about 20 mM proline. In a preferred embodiment, the lysis buffer comprises between about 2 mM and about 10 mM arginine and between about 2 mM and about 10 mM proline. In a most preferred embodiment, the lysis buffer comprises between about 3.5 mM and about 6.5 mM arginine and between about 3.5 mM and about 6.5 mM proline.

The lysis buffer according to the kits and methods of the invention further comprises a calcium salt. In a preferred embodiment, the calcium salt is CaCl₂. In one embodiment, the concentration of CaCl₂ in the lysis buffer is between about 0.05 mM and about 1.5 mM. In a preferred embodiment, the concentration of CaCl₂ in the lysis buffer is between about 0.1 mM and about 0.5 mM. In a most preferred embodiment, the concentration of CaCl₂ in the lysis buffer is between about 0.15 mM and about 0.35 mM.

In certain embodiments, the non-ionic non-denaturing detergent in the lysis buffer according to the kits and methods of the invention may be one selected from the group consisting of Nonidet^{™} P40, IGEPAL^{®} CA-630, Triton^{™}, Tween^{®} 20 or any of their substitutes. Preferably, the non-ionic non-denaturing detergent in the lysis buffer is Nonidet™ P40 or IGEPAL^{®} CA-630. The detergents are selected in an amount so as to render the cell membranes permeable so that agents can enter the cell cytoplasmic domain and RNA can exit the cell cytoplasmic domain. In one embodiment, the non-ionic non-denaturing detergent is present in the lysis buffer at a concentration between about 0.01% and about 0.5% by volume. In one embodiment, the non-ionic non-denaturing detergent in the lysis buffer is Nonidet™ P40 or IGEPAL^{®} CA-630 at a concentration between about 0.01% and about 0.5% by volume. In a preferred embodiment, the non-ionic non-denaturing detergent in the lysis buffer is Nonidet^{™} P40 or IGEPAL^{®}CA-630 at a concentration between about 0.02% and about 0.2% by volume.

In certain embodiments, the PEG in the lysis buffer according to the kits and methods of the invention may be any PEG of molecular weight between 1000 and 20000, such as PEG 3000, PEG 4000, PEG 6000, PEG 6000, PEG 8000, PEG 10000, PEG 12000, PEG 15000, and PEG 20000. In one embodiment, the PEG in the lysis buffer has a molecular weight between 4000 and 8000, most preferably around 6000.

In a further embodiment, the concentration of PEG in the lysis buffer according to the kits and methods of the invention is between 0.01 % and 1% by volume. In one embodiment, the concentration of PEG of molecular weight 4000 to 8000 in the lysis buffer is between 0.05 % and 0.2% by volume. In a preferred embodiment, the lysis buffer comprises PEG of molecular weight 4000 to 8000 at a concentration between 0.01 % and 1% by volume. In a more preferred embodiment, the lysis buffer comprises PEG of molecular weight 4000 to 8000 at a concentration between 0.05 % and 0.2% by volume. In a most preferred embodiment, the lysis buffer comprises PEG of molecular weight around 6000 at a concentration between 0.05 % and 0.2% by volume.

In preferred embodiments, the lysis buffer according to the kits and methods of the invention advantageously does not comprise a chaotropic salt.

In certain embodiments, further additives can be incorporated into the lysis buffer according to the kits and methods of the invention, e.g. DNase and/or RNase inhibitors can be used that protect released nucleic acids from degradation. Suitable inhibitors and other protective agents are known in the prior art and, thus, do not need any detailed description here.

An antibacterial agent, e.g., sodium azide, may be included in the lysis buffer according to the kits and methods of the invention to extend the shelf-life of the lysis buffer. The amount of antibacterial agent depends on the agent and the storage conditions and should be selected so as not to interfere with the extraction process but to provide the desired shelf-life. Any antibacterial agent that extends shelf-life without unduly degrading the quality of the RNA obtained is therefore suitable for use in the lysis buffer of the invention. According to alternative kit and method aspects of the invention, the lysis buffer according to any of the embodiments described hereinabove may also comprise a heat-metastable protease, as explained in sections II (kits) and III (methods). In a preferred embodiment, the lysis buffer described hereinabove does not contain a heat-metastable protease.

As will be evident from the Examples, the lysis buffer according to the kits and methods of the invention used together with a heat-metastable protease was surprisingly effective in extracting intact RNA from a number of different samples, including complex samples. Without wishing to be bound by theory, it is believed that the advantages of the kits and methods of the invention can be traced to the protease-enhancing properties of the particular lysis buffer, together with the protein (i.e. RNase)-degrading properties of the heat-metastable protease which is added to it.

The lysis buffer according to the kits and methods of the invention is therefore suitable for use in a range of methods of RNA extraction, as well as in a range of kits for RNA extraction.

### EXAMPLES

The following examples investigated RNA extraction by adjusting various parameters such as temperature, buffer composition and protease addition and amount. In the experiments of the examples, two buffers termed "Buffer A" and "Buffer B" are used. Buffer A is a buffer which is representative of lysis buffers used in prior art kits and methods to extract RNA from whole cells or tissues. The compositions of these buffers are as follows:
Buffer A:
   50 mM Tris/HCl pH 8.5
   1 mM EDTA
   0.45% (v/v) Nonidet™ P40
   0.45% (v/v) Tween^{®}20
   0.1% (v/v) Polyvinylpyrrolidone, MW 10,000
Buffer B:
   6 mM HEPES pH 7.9
   0.05 mM EDTA Tritriplex III
   0.06% (v/v) Nonidet™ P40
   0.1%(v/v)PEG MW 6,000
   0.25 mM CaCl₂
   5 mM Arginine
   5 mM Proline

### Example 1: RNA can be extracted from a lysed sample as long as inherent RNase activity is low

### Experiment 1

This section shows that viral RNA can be extracted from a biological sample as long as the sample matrix is almost free of inherent RNase activity.

### Setup:

RNA was extracted from a culture supernatant containing Bacteriophage fr. The procedure was as follows:
100µl Buffer A was spiked with 1µl culture supernatant. This was mixed with 10µl magnetic particles. The mixture was shaken with 1400rpm for 5min at 25, 40, 60, 80, and 95°C. Then, the magnetic particles were separated from the mixture by putting the tubes into a magnetic stand for 1min (reverse purification). 100µl of the remaining supernatant was transferred into a new microtube.

RNA content of the extracts was quantified using an RT-qPCR assay specific to Bacteriophage fr.

### Results:

Table 1 shows that according to this procedure RNA could be extracted for all samples. The amount of RNA extracted increased with increasing temperature, with the highest yield at 95°C.

**Table 1: Ct-values from Phage fr-specific RT-qPCR using the RNA extracts prepared as described above.**

| **Condition** | **mean Ct** | **std.dev Ct** | **extraction efficiency** |
|---|---|---|---|
| 25°C | 24.32 | 0.04 | 5% |
| 40°C | 21.77 | n.a. | 29% |
| 60°C | 20.53 | 0.12 | 68% |
| 80°C | 20.54 | 0 | 67% |
| 95°C | 19.96 | 0.08 | 100% |

### Experiment 2

### Setup:

Here, another sample matrix was tested: throat swabs eluted in PBS and spiked with inactivated Influenza virus. RNA from these samples was prepared as follows:
0.3ml spiked throat eluate was mixed with 0.6ml 30mM sodium acetate pH 5.5 and 30µl magnetic particles. The mixture was shaken with 1400rpm at room temperature for 5min. Then, magnetic particles were separated by putting the tubes into a magnetic stand for 1 min. The supernatant was discarded, and then the magnetic particles were resuspended in 200µl 30mM sodium acetate pH 5.5, and separated again by incubation on a magnetic stand for 1min (enrichment procedure). Again, the supernatant was discarded, the particles were resuspended in 150µl Buffer A and incubated for 10 min at 95°C with shaking at 1400 rpm. Afterwards, condensed liquid on the lid of the tube was removed by a short centrifuge spin and the particles were separated using a magnetic stand again (see above). 100µl of the supernatant was transferred into a new microtube.

RNA content of the extract was quantified using the QIAGEN artus Influenza H1N1 PCR Kit according to the manufacturer's instructions, which uses primers for influenza amplification as follows:

| | |
|---|---|
| FWD | CTTCTGATCCGCATAGTGACGAC |
| REV | AACGGTCATTCGCCTCCAGCAG |
| PROBE | TAGGGGATGGTAACGACGAAGCA |

### Results:

Table 2 shows that according to this procedure RNA could be extracted for all samples.

**Table 2: Ct-values from Influenza-specific RT-qPCR using the RNA extracts prepared as described above.**

| **sample prep** | **Ct value** |
|---|---|
| #1 | 15.45 |
| #2 | 15.76 |
| #3 | 17.90 |
| #4 | 15.78 |
| mean | 16.22 |
| std.dev. | 1.13 |

### Example 2: RNA cannot be extracted from a lysed sample if the sample is blood plasma or nasal swabs

### Experiment 3

Experiments 1 and 2 showed that it is possible to extract intact RNA using Buffer A (and reverse purification methodology). This experiment demonstrates the devastating effect of RNases inherent in certain sample matrices on viral RNA.

### Setup:

A panel of samples was generated by mixing Buffer A with nasal swab material eluted in Buffer A according to Table 3.

**Table 3: Panel of mixtures prepared for this experiment.**

| **Mixtures** | **neat Buffer A [µl]** | **nasal swab in Buffer A [µl]** | **% of nasal specimen** |
|---|---|---|---|
| #1 | 900 | 0 | 0% |
| #2 | 891 | 9 | 1% |
| #3 | 873 | 27 | 3% |
| #4 | 810 | 90 | 10% |
| #5 | 630 | 270 | 30% |
| #6 | 0 | 900 | 100% |

The mixtures 1 to 6 were spiked with purified RNA from Bacteriophage fr and mixed well. Then these final samples have been treated as follows:
150µl spiked mixture (#1 - #6) was mixed with 10µl magnetic particles. These mixtures were shaken with 1400rpm for 5min at 25, 60, 80 and 95°C. Then, magnetic particles were separated by putting the tubes into a magnetic stand for 1 min. 100µl of the supernatant was transferred into new microtubes.

RNA content of the extracts was quantified using an RT-qPCR assay specific to Bacteriophage fr.

### Results:

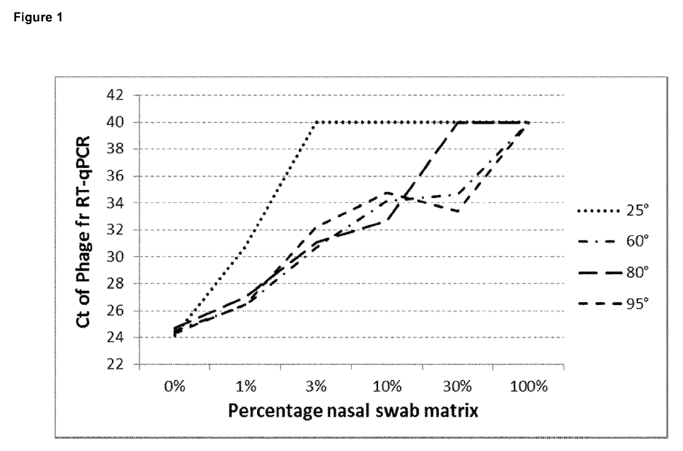

Figure 1 of the application shows increasing Ct values for fr-specific RT-qPCRs (corresponding to decreasing amount of RNA) with increasing percentage of nasal swab specimen in the mixture. Samples treated with 25°C contained no detectable RNA even at 3% nasal swab content whereas samples treated with higher temperatures reached this level at a much higher nasal swab content.

### Conclusion:

The example demonstrates that certain biological specimens (in this experiment nasal swab material was chosen) contain a high amount of RNases whose activity efficiently degrades RNA. With only 3% nasal swab content the Ct drifts about 7 cycles. This corresponds to a reduction of RNA by 99%. Experiment 3 also showed that incubation at elevated temperatures could not prevent this effect.

### Example 3: The addition of a heat-metastable protease to buffer A does not reduce the RNA yield

### Experiment 4

Based on the results from Experiment 3, it was investigated how to eliminate RNase activity originating from the biological specimen. This is necessary to preserve the heat-extracted RNA, because the naturally occurring RNase enzymes obviously survive the heat-extraction procedure and remain active after cooling down of the RNA extract. This is in line with common knowledge that RNases are extremely stable proteins, which can easily refold after a denaturation event [Mui et al. (1985) Biochemistry 24(16):4481-9].

To test whether it could eliminate RNase activity, a heat-metastable protease, subtilisin, was added to the lysis buffer A.

### Setup:

Neat Buffer A and a nasal swab eluted in Buffer A were spiked with Bacteriophage fr. Viral RNA was extracted as follows:
100µl sample was supplemented with 1µl subtilisin (which had a specific activity of 530 mAU/ml) giving a concentration of 5.2 mAU/ml, and magnetic particles in different combinations as follows:

| | | |
|---|---|---|
| 100µl Buffer A | + 10µl Magnetic particles | |
| 100µl Buffer A | + 10µl Magnetic particles | + 1µl subtilisin |
| 100µl Nasal swab eluate/Buffer A | + 10µl Magnetic particles | |
| 100µl Nasal swab eluate/Buffer A | + 10µl Magnetic particles | + 1µl subtilisin |

The mixture was shaken with 1400rpm at 95°C for 5min. Afterwards, the mixtures were rapidly cooled on ice and the particles were separated in a magnetic stand for 1 min. 100µl of the supernatant was transferred into a new microtube.

RNA content of the extracts was quantified using RT-qPCR assay specific to Bacteriophage fr.

### Results:

The results are shown in Table 4.

**Table 4: Ct-values from Bacteriophage fr-specific RT-qPCRs using aliquots of the samples treated as described above.**

| **Spiked sample** | **Prep condition** | **mean Ct** | **std.dev Ct** |
|---|---|---|---|
| neat Buffer A | Magnetic particles w/o subtilisin | 23.87 | 0.19 |
| neat Buffer A | Magnetic particles +5.2 mAU/ml subtilisin | 23.82 | 0.39 |
| nasal swab eluate / Buffer A | Magnetic particles w/o subtilisin | 26.57 | 0.21 |
| nasal swab eluate / Buffer A | Magnetic particles +5.2 mAU/ml subtilisin | n.d. | n.a |

The results in Table 4 again indicate that buffer A is not very efficient in extracting RNA from a sample with high inherent RNAse activity (delta Ct of 2.3 for spiked nasal swab vs clean virus in buffer A). Furthermore, the RNA yield in this case is not improved by addition of subtilisin to buffer A.

However, the experiment shows that the addition of 5.2 mAU/ml subtilisin to buffer A is not detrimental to the RNA yield (almost identical Cts for neat buffer A +/- subtilisin).

### Example 4: Exchange of Buffer A with Buffer B improves the integrity of extracted RNA

### Experiment 5

In this experiment different lysis buffers, A and B, were compared with each other in the RNA extraction procedure.

### Setup:

Blood plasma was spiked with Influenza A viruses and incubated for 5min at room temperature. Then, viral RNA was extracted as follows:
100µl spiked blood plasma was mixed with 200µl enrichment buffer and 10µl magnetic particles. The mixture was incubated for 3min at room temperature and then the magnetic particles were separated in a magnetic stand for 1 min. The supernatant was discarded and the magnetic particles were resuspended in 100µl Buffer A or Buffer B. Both conditions were processed in two ways: with and without addition of 1µl subtilisin (which had a specific activity of 530 mAU/ml), giving a concentration in the mixture of 5.2 mAU/ml. The mixtures were shaken with 1400rpm at 95°C for 5 min. Afterwards, the mixtures were rapidly cooled on ice and the particles were separated in a magnetic stand for 1 min. 80µl of the supernatant was transferred into a new microtube.

RNA content of the extracts was quantified using an RT-qPCR assay specific to Influenza A.

### Results:

Table 5 shows that even the procedure using Buffer A yields considerable amounts of Influenza A RNA. However, the use of Buffer B improved the RNA yield by 2.5 Ct's. The addition of subtilisin in both buffers increased the RNA yield significantly.

**Table 5: Ct-values from Influenza A-specific RT-qPCRs using aliquots of the samples treated as described above.**

| **Condition** | **mean Ct** | **std.dev Ct** |
|---|---|---|
| Buffer A, no subtilisin | 32.71 | 0 |
| Buffer A, 5.2 mAU/ml subtilisin | 28.36 | 0.15 |
| Buffer B, no subtilisin | 29.24 | 0.28 |
| Buffer B, 5.2 mAU/ml subtilisin | 25.81 | 0.82 |

The fact that in this experiment even the Buffer A procedure with and without subtilisin yielded detectable amounts of viral RNA may be attributed to the enrichment step upfront of the lysis step, because this enrichment potentially led to a removal of a large part of the RNases inherent to blood plasma.

In further experiments it could be repeated that the RNA yield of the procedure using Buffer B is consistently much higher than the yield of the procedure using Buffer A.

### Example 5: RNA extraction yield depends on the amount of protease added

When using proteases to digest RNases within certain specimen, care needs to be taken about both the type and amount of protease added to the lysis sample. This is because some proteases are more heat-stable than others, and residual protease activity that survives the heat lysis step can impair the downstream nucleic acid amplification process by inactivating amplification enzymes.

### Experiment 6

In this experiment, the amount of subtilisin added to the lysis buffer was titrated and the overall effect on RNA yield was examined in terms of the Ct of RT-PCR amplification reactions.

### Setup:

A nasal swab was taken and eluted in Buffer A. This solution was spiked with Influenza A virus and incubated for 5min at room temperature. Then, viral RNA was extracted as follows:
100µl spiked nasal swab eluate was mixed with 200µl enrichment buffer and 10µl magnetic particles. The mixture was incubated for 3min at room temperature and then magnetic particles were separated in a magnetic stand for 1min. The supernatant was discarded and the magnetic particles were resuspended in 100µl Buffer B and increasing amounts (from 0µl up to 2µl) of subtilisin which had a specific activity of 530 mAU/ml. The mixtures were shaken with 1400rpm at 95°C for 5min. Afterwards, the mixtures were rapidly cooled on ice and the particles were separated in a magnetic stand for 1 min. 80µl of the supernatant was transferred into a new microtube.

RNA content of the extracts was quantified using RT-qPCR assay specific to Influenza A.

### Results:

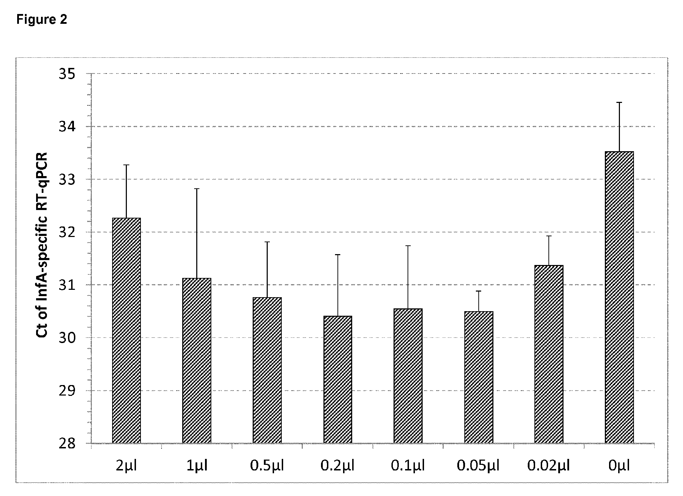

Table 6 and Figure 2 of the application show that the addition of heat-metastable protease (subtilisin) greatly improves the RNA yield compared to no protease added. Simultaneously, the data also show that addition of too much heat-metastable protease is counter-productive and leads to a loss of RNA yield back to the region of "no protease added". The optimum range of heat-metastable protease in the lysis buffer is around 0.2 to 4.0 mAU/ml.

**Table 6: Ct-values from Influenza A-specific RT-qPCRs using aliquots of the samples treated as described above.**

| **Condition** | **Subtilisin concentration in lysis buffer (mAU/ml)** | **mean Ct** | **std.dev Ct** |
|---|---|---|---|
| Buffer B + magnetic particles + 2µl subtilisin | 10.4 | 32.27 | 1.00 |
| Buffer B + magnetic particles + 1µl subtilisin | 5.2 | 31.13 | 1.69 |
| Buffer B + magnetic particles + 0.5µl subtilisin | 2.6 | 30.77 | 1.05 |
| Buffer B + magnetic particles + 0.2µl subtilisin | 1.1 | 30.41 | 1.16 |
| Buffer B + magnetic particles + 0.1µl subtilisin | 0.53 | 30.55 | 1.19 |
| Buffer B + magnetic particles + 0.05µl subtilisin | 0.26 | 30.50 | 0.38 |
| Buffer B + magnetic particles + 0.02µl subtilisin | 0.11 | 31.37 | 0.55 |
| Buffer B + magnetic particles + 0µl subtilisin | 0 | 33.52 | 0.93 |

Those skilled in the art will recognize, or be able to ascertain using no more than routine experimentation, numerous equivalents to the specific embodiments described herein both in the Examples and in the body of the entire patent description. Such equivalents are considered to be within the scope of this invention and are intended to be encompassed by the following claims.

## Claims

1. A kit for extracting RNA from an RNA-containing sample, comprising:
(a) a lysis buffer comprising a zwitterionic buffering agent, a chelating agent, a basic amino acid, a cyclic amino acid, a calcium salt, a non-ionic non-denaturing detergent, and polyethylene glycol (PEG); and
(b) a heat-metastable protease.

2. The kit of claim 1, wherein the kit further comprises one or more of the following:
(c) magnetic particles for performing sample enrichment and/or reverse purification of RNA;
(d) reagents (e.g. wash buffers, elution buffers) for performing sample enrichment, and/or column purification or reverse purification of RNA;
(e) deoxyribonuclease; and/or
(f) an instruction manual detailing how to use the kit to extract RNA.

3. Method of extracting RNA from an RNA-containing sample, comprising the following steps:
(i) adding a heat-metastable protease to a lysis buffer, wherein the lysis buffer comprises a zwitterionic buffering agent, a chelating agent, a basic amino acid, a cyclic amino acid, a calcium salt, a non-ionic non-denaturing detergent, and polyethylene glycol (PEG);
(ii) contacting the RNA-containing sample with the lysis buffer containing the heat-metastable protease of step (i) to form a mixture; and
(iii) optionally heating the mixture to at least 60°C, at least 70°C or at least 80°C.

4. Method of extracting RNA from an RNA-containing sample, comprising the following steps:
(i) contacting the RNA-containing sample with a lysis buffer to form a mixture, wherein the lysis buffer comprises a zwitterionic buffering agent, a chelating agent, a basic amino acid, a cyclic amino acid, a calcium salt, a non-ionic non-denaturing detergent, polyethylene glycol (PEG), and a heat-metastable protease; and
(ii) optionally heating the mixture to at least 60°C, at least 70°C or at least 80°C.

5. The kit of claims 1 or 2, or the method of claims 3 or 4,
wherein the lysis buffer comprises HEPES or MOPS as the zwitterionic buffering agent, EDTA or EGTA as the chelating agent, arginine as the basic amino acid, proline as the cyclic amino acid, CaCl₂ as the calcium salt, one of Nonidet P40, IGEPAL CA-630, Triton or Tween20 as the non-ionic non-denaturing detergent, and wherein the polyethylene glycol (PEG) of the lysis buffer has a molecular weight between 4000 and 8000.

6. The kit of any one of claims 1, 2 or 5, or the method of any one of claims 3-5, wherein the lysis buffer comprises:
(a) HEPES pH 7.5 to 8.5;
(b) Disodium EDTA;
(c) arginine;
(d) proline;
(e) CaCl₂;
(f) Nonidet P40 or IGEPAL CA-630; and
(g) PEG of molecular weight around 6000.

7. The kit of any one of claims 1, 2, 5 or 6, or the method of any one of claims 3-6, wherein the RNA-containing sample
(a) is a swab, optionally a throat swab or a nasal swab;
(b) comprises whole blood, blood plasma or blood serum;
(c) comprises cultured cells, optionally infected with virus particles;
(d) is a nasal wash or a nasal aspirate;
(e) is a broncho-alveolar lavage (BAL);
(f) is a tissue sample (e.g. epithelial, buccal, cervical, intestinal, pancreas, muscle, kidney, heart, lung, brain, bone, prostate, skin, hair, or a removed tumour tissue sample);
(g) is a bodily fluid (e.g. sputum, saliva, tears, cerebrospinal fluid (CSF), urine, stool, semen, or sweat); or
(h) is a liquor sample (e.g. from a lumbar puncture).

8. The method of claim 7 (b), (c), (d), (e), (g) or (h), wherein prior to the lysis step the sample is enriched for the presence of biological target material (e.g. cells, viruses, bacteria).

9. The method of claim 8, wherein the enrichment procedure
(a) employs magnetic particles which bind to said biological target material in the sample, and wherein the magnetic particles bound to biological target material are subsequently removed from the sample by applying an external magnetic force; and/or
(b) does not employ a precipitating agent.

10. The kit of any one of claims 1, 2 or 5-7, or the method of any one of claims 3-9, wherein the heat-metastable protease is added to the lysis buffer in an amount which is sufficient to inactivate RNAses in the mixture that forms when contacting the sample with the lysis buffer, but which does not impair amplification enzymes which are added to the mixture in downstream applications.

11. The kit of any one of claims 1, 2, 5-7 or 10, or the method of any one of claims 3-10, wherein the heat-metastable protease is added to the lysis buffer to provide a concentration in the lysis buffer between 0.2 and 4.0 mAU/ml.

12. The kit of any one of claims 1, 2, 5-7, 10 or 11, or the method of any one of claims 3-11, wherein the heat-metastable protease is subtilisin or proteinase K, preferably subtilisin.

13. The kit of any one of claims 2, 5-7 or 10-12, or the method of any one of claims 3-12, wherein deoxyribonuclease is also added to the lysis buffer prior to contacting the sample with the lysis buffer.

14. The method of any one of claims 3-13, wherein the RNA is recovered from the mixture by column purification or by reverse purification.

15. The method of claim 14, wherein the reverse purification employs magnetic particles which bind to non-RNA components in the mixture, wherein the magnetic particles bound to non-RNA components are subsequently removed from the mixture by applying an external magnetic force.
